(19) Europäisches Patentamt
European Patent Office
Office européen des brevets

(11) Publication number: **0 017 411**
**B1**

(12) # EUROPEAN PATENT SPECIFICATION

(45) Date of publication of patent specification: **31.03.82**

(51) Int. Cl.³: **C 07 D 401/04,**
A 61 K 31/495

(21) Application number: **80300909.1**

(22) Date of filing: **24.03.80**

(54) **Phthalazine cardiac stimulants, processes for preparing them, and pharmaceutical compositions containing them.**

(30) Priority: **28.03.79 GB 7910843**

(43) Date of publication of application:
**15.10.80 Bulletin 80/21**

(45) Publication of the grant of the patent:
**31.03.82 Bulletin 82/13**

(84) Designated Contracting States:
**AT BE CH DE FR GB IT LU NL SE**

(56) References cited:
**GB - A - 2 000 136**
**GB - A - 2 007 654**
**GB - A - 2 008 104**

(73) Proprietor: **Pfizer Limited**
**Ramsgate Road**
**Sandwich Kent CT13 9NJ (GB)**

(84) **GB**

(73) Proprietor: **Pfizer Corporation**
**Calle 15 1/2 Avenida Santa Isabel**
**Colon (PA)**

(84) **BE CH DE FR IT LU NL SE AT**

(72) Inventor: **Campbell, Simon Fraser**
**Grey Friars Upper Street Kingsdown**
**Deal Kent (GB)**
Inventor: **Danilewicz, John Christopher**
**44 Sandwich Road**
**Ash, Nr. Canterbury Kent (GB)**
Inventor: **Ham, Allan Leslie**
**84 Northwood Road**
**Broadstairs, Kent (GB)**
Inventor: **Stubbs, John Kendrick**
**111 Blemheim Road**
**Deal, Kent (GB)**

(74) Representative: **Wood, David John et al,**
**Pfizer Limited Ramsgate Road**
**Sandwich Kent CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 017 411 B1**

# Phthalazine cardiac stimulants, processes for preparing them, and pharmaceutical compositions containing them

This invention relates to therapeutic agents which are novel derivatives of phthalazine, and is particularly concerned with such derivatives having a substituted piperidino group in the 1-position. Certain 6,7-dialkoxy-1-(substituted piperidino)phthalazine cardiac stimulants are disclosed in our published UK Patent Application No. 2 000 136 A.

The compounds of the invention are phosphodiesterase inhibitors and cardiac stimulants of which a preferred class selectively increase the force of myocardial contraction without producing significant increases in heart rate. The compounds are useful in the curative or prophylactic treatment of cardiac conditions, in particular heart failure.

According to the invention there are provided novel phthalazine compounds of the formula:—

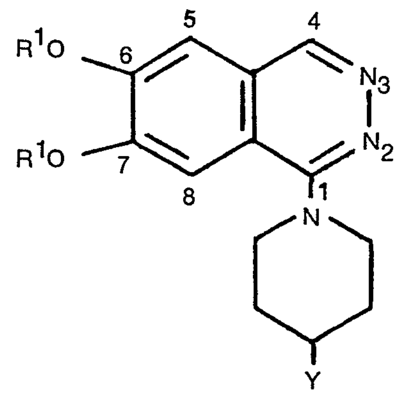

(I)

wherein

$R^4$ is a $C_1$—$C_4$ alkyl group; and Y is a group of the formula: $-(CH_2)_m-Z$

wherein

m is 1 or 2; and Z is a group selected from:

$-OCONR^3R^4$,
$-N(R^2)COR^3$,
$-N(R^2)SO_2R^3$,
$-N(R^2)CONR^3R^4$,
$-N(R^2)SO_2NR^3R^4$,
and $-N(R^2)COOR^3$,

wherein

$R^2$ and $R^4$ are each independently hydrogen or $C_1$—$C_4$ alkyl, and $R^3$ is $C_3$—$C_7$ cycloalkyl; and the pharmaceutically acceptable acid addition salts thereof.

Where appropriate, the alkyl groups may be straight or branched chain.

The pharmaceutically acceptable acid addition salts of the compounds of the invention are those formed from acids which form non-toxic acid addition salts containing pharmaceutically acceptable anions, such as the hydrochloride, hydrobromide, hydroiodide, sulphate or bisulphate, phosphate or acid phosphate, acetate, maleate, fumarate, lactate, tartrate, citrate, gluconate, saccharate and p-toluenesulphonate salts.

The cardiac stimulant activity of the compounds of the invention is shown by their effectiveness in one or more of the following tests: (a) increasing the force of contraction in the isolated, spontaneously beating, guinea pig double atria or kitten right and left atria preparations; (b) increasing myocardial contractility (left ventricular $dP/_{dt}max.$) in the anaesthetised cat or dog with a left ventricular catheter; (c) increasing myocardial contractility in the conscious dog with an implanted left ventricular transducer.

In test (a) the positive inotropic and chronotropic responses of the atria to test compound are measured at several doses and compared with the responses elicited by isoprenaline. The comparison of the dose response curves obtained gives a measure of the force versus rate selectivity of the test compound.

In test (b) the positive inotropic action of the test compound following intravenous administration is measured in the anaesthetised cat or dog. The magnitude and duration of this action, and the selectivity for increase in force versus frequency of contraction of the test compound are obtained, as are its peripheral effects, e.g. the effect on the blood pressure.

In test (c) the positive inotropic action of the test compound following intravenous or oral administration to a conscious dog with an implanted left ventricular transducer is measured. The magnitude of the inotropic action, the selectivity for increase in force versus frequency of contraction, and the duration of action of the inotropic effect of the test compound are all obtained.

The preferred compounds of the invention have the formula:

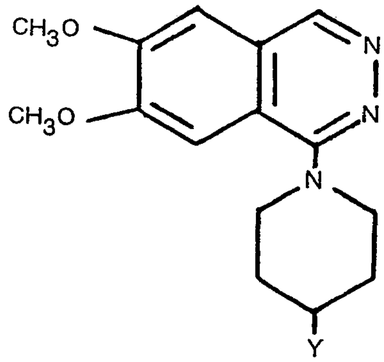

(II)

wherein Y is as defined for formula (I). Preferably $R^2$ and $R^4$ are each independently hydrogen or methyl. Preferably $R^3$ is a $C_3$—$C_6$ cycloalkyl group.

Z is preferably selected from the following:—

(a) —O.CON($R^4$) (cyclohexyl) wherein $R^4$ is H or $CH_3$;
(b) —N($CH_3$)$COR^3$ wherein $R^3$ is $C_3$—$C_6$ cycloalkyl;
(c) —N($CH_3$)$SO_2R^3$ wherein $R^3$ is cyclopropyl, cyclopentyl or cyclohexyl;
(d) —N($CH_3$)$CONR^3R^4$ wherein $R^3$ is cyclopentyl or cyclohexyl and $R^4$ is H or $CH_3$;
(e) —N($CH_3$)$SO_2NH.R^3$ wherein $R^3$ is cyclopropyl or cyclohexyl;
*and*
(f) —N($R^2$)COO.cyclohexyl wherein $R^2$ is H or $CH_3$.

In the preferred individual compounds, $R^1$ is $CH_3$ and Y is selected from —$CH_2CH_2N(CH_3)CON(CH_3)$-cyclopentyl, —$CH_2CH_2N(CH_3)CO$.cyclopentyl and —$CH_2CH_2N(CH_3)SO_2NH$.cyclohexyl.

The compounds of the invention can be administered alone but will generally be administered in admixture with a pharmaceutical carrier selected with regard to the intended route of administration and standard pharmaceutical practice. For example they may be administered orally in the form of tablets containing such excipients as starch or lactose, or in capsules either alone or in admixture with excipients, or in the form of elixirs or suspensions containing flavouring or colouring agents. They may be injected parenterally, for example, intravenously, intramuscularly or subcutaneously. For parenteral administration, they are best used in the form of a sterile aqueous solution which may contain other solutes, for example, enough salts or glucose to make the solution isotonic.

For administration to man in the curative or prophylactic treatment of cardiac conditions such as congestive heart failure, it is expected that oral dosages of the most active compounds of the invention will be in the range from 20 mg to 1 g daily, taken in 2 to 4 divided doses per day, for an average adult patient (70 kg). Dosages for intravenous administration would be expected to be within the range of 1 to 300 mg per single dose as required, for example in the treatment of acute heart failure. Thus for a typical adult patient, individual tablets or capsules might contain from 5 to 250 mg of active compound, in a suitable pharmaceutically acceptable vehicle or carrier.

Thus the present invention provides a pharmaceutical composition comprising a compound of the formula (I) as defined above or pharmaceutically acceptable acid addition salt thereof together with a pharmaceutically acceptable diluent or carrier.

The invention yet further provides a compound of the formula (I) or pharmaceutically acceptable acid addition salt thereof, for use in treating an animal, including a human being, to stimulate the heart of the animal.

The compounds of the invention may be prepared by a number of routes:

*Route A*

Compounds of the formula (I) may be prepared by reacting an appropriately substituted phthalazine of the formula:

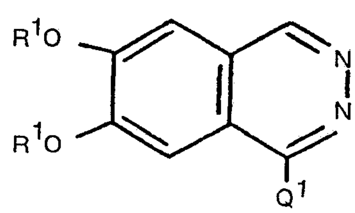

(III)

wherein $Q^1$ represents a facile leaving group such as chloro-, bromo-, iodo-, lower alkoxy or (lower alkyl)thio, with an amine of the formula:

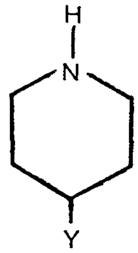

(IV)

with resultant elimination of $HQ^1$. $Q^1$ is preferably chloro or bromo.

In a typical procedure the phthalazine (III) and piperidine (IV) are heated together in a suitable solvent, e.g. ethanol, in the presence of a base such as triethylamine for up to about 72 hours. The mixture may then be concentrated *in vacuo*, the residue dissolved in a suitable solvent, e.g. chloroform, washed with water, dried and concentrated *in vacuo*. The resulting oil in a suitable solvent, e.g. ethyl acetate, can then be chromatographed on e.g. a silica column, the column being eluted with e.g. ethylacetate/5% ether then ethyl acetate. Appropriate fractions are collected, concentrated *in vacuo*, and e.g. the oxalate salt precipitated by the addition of excess oxalic acid in isopropanol to a solution of the crude product in the same solvent. The salt may be recrystallised to purity from a suitable solvent, e.g. isopropanol.

The starting materials of the formulae (III) and (IV) are either known compounds or may be prepared by procedures analogous to those of the prior art, e.g. as follows:—

(a)

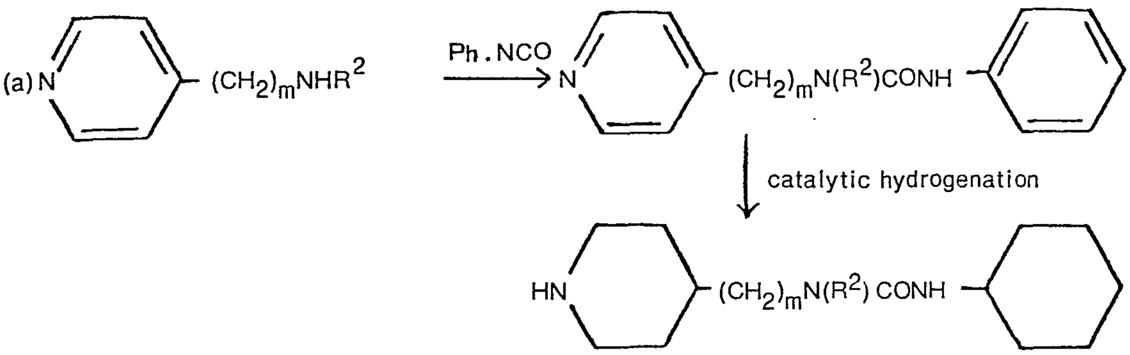


This process can also be carried out using an isocyanate of the formula $R^3.NCO$ in place of phenyl isocyanate.

(b)

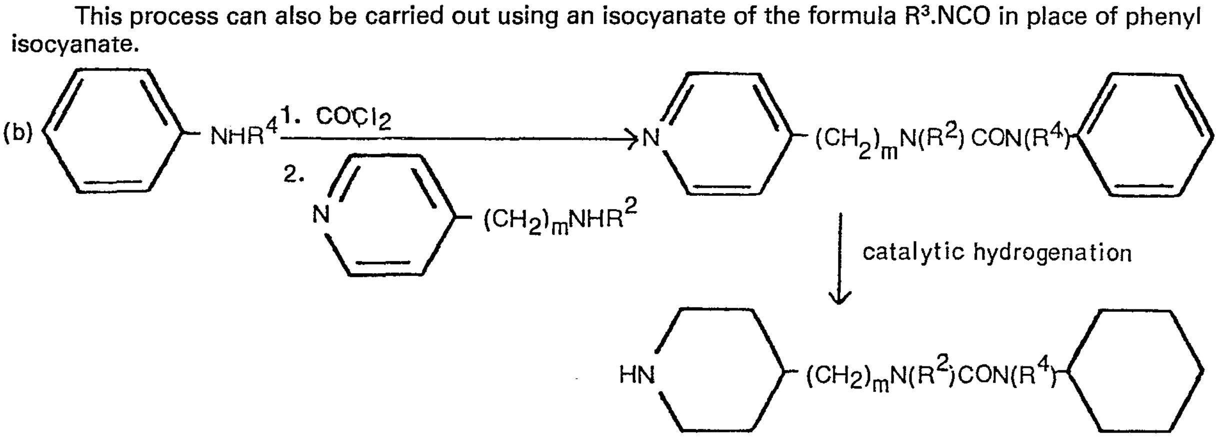


This process can also be carried out using an amine of the formula $R^3R^4NH$ in place of $Ph.NHR^4$. 1,1′-Carbonyldiimidazole may be used in place of phosgene.

(c) $R^3COOH$

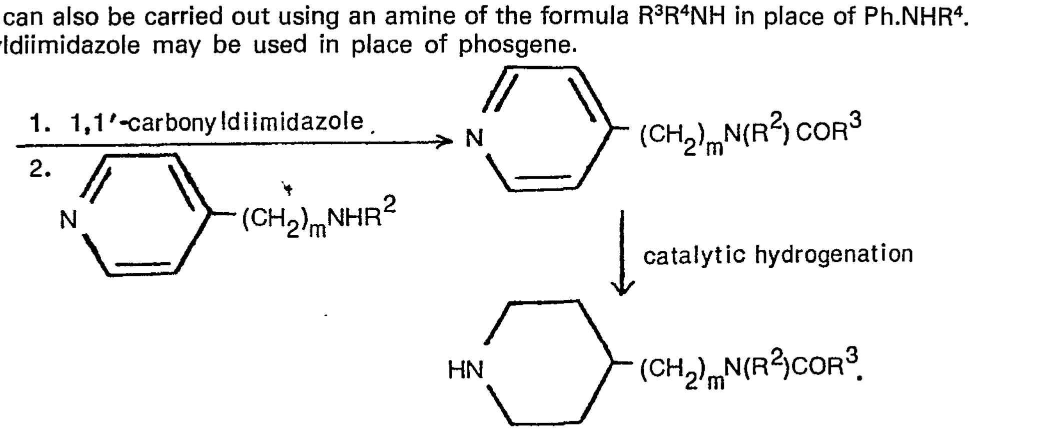

(d) 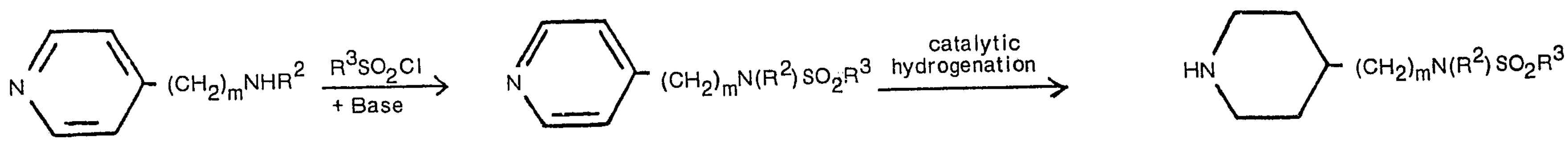
R3SO2Cl
+ Base
catalytic
hydrogenation

(e) 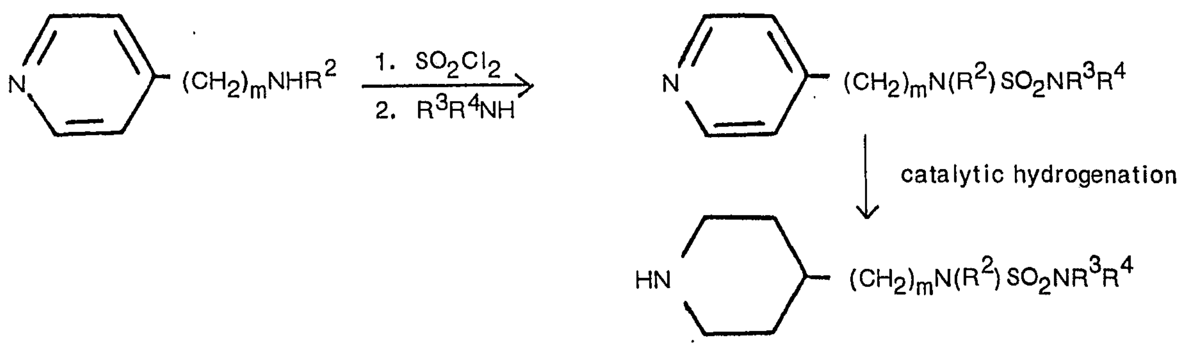
1. SO2Cl2
2. R3R4NH
catalytic hydrogenation

(f) 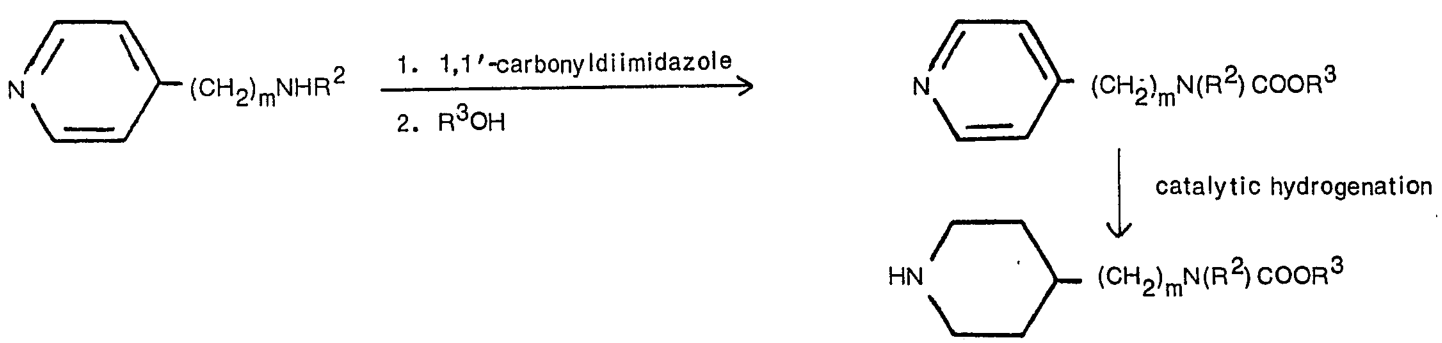
1. 1,1'-carbonyldiimidazole
2. R3OH
catalytic hydrogenation

(g)

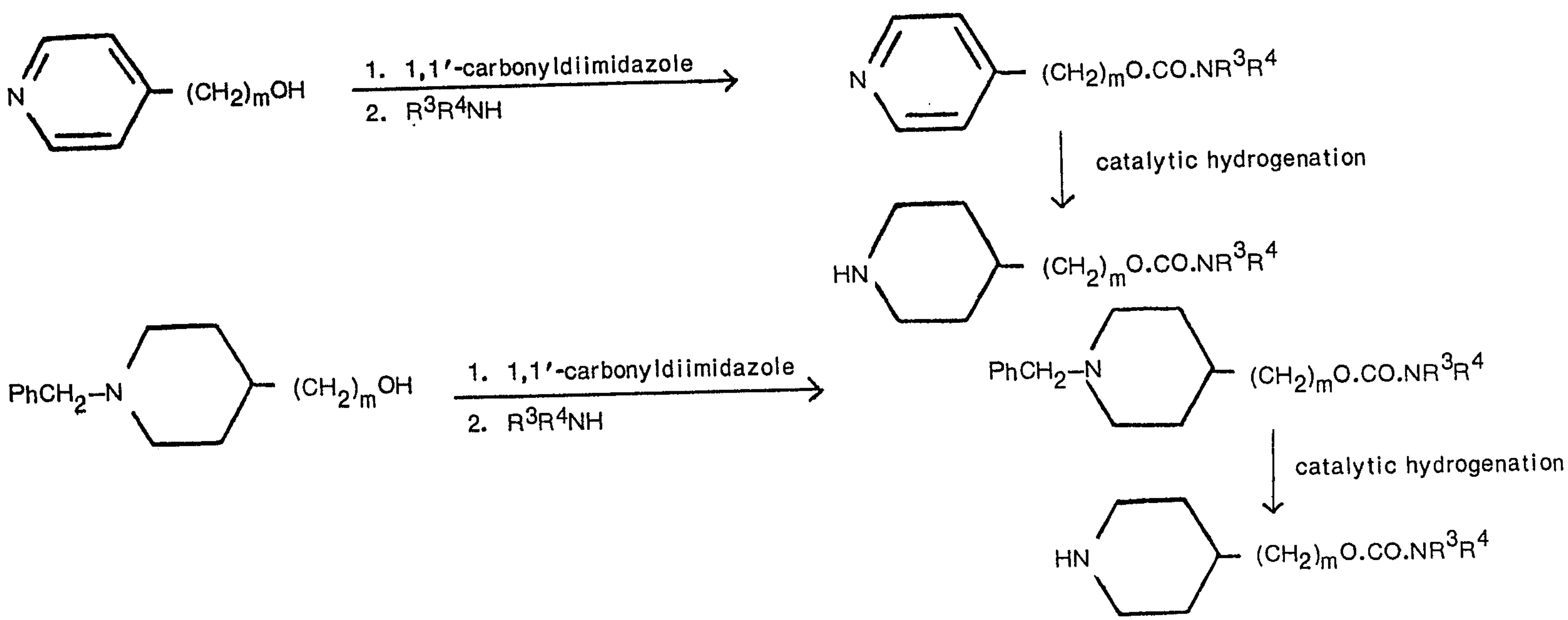
1. 1,1'-carbonyldiimidazole
2. R3R4NH
catalytic hydrogenation
1. 1,1'-carbonyldiimidazole
2. R3R4NH
catalytic hydrogenation

or

(h)

The preparation of 1-chloro-6,7-dimethoxyphthalazine is described in Preparation 3 of published British Patent Application No. 2000136A.

Routes (a) to (h) are above are illustrated in detail in the Preparations hereinafter.

*Route B*

Compounds of the formula (I) in which Y is $-(CH_2)_mN(R^2)CONHR^3$ may be prepared by reacting a phthalazine of the formula:—

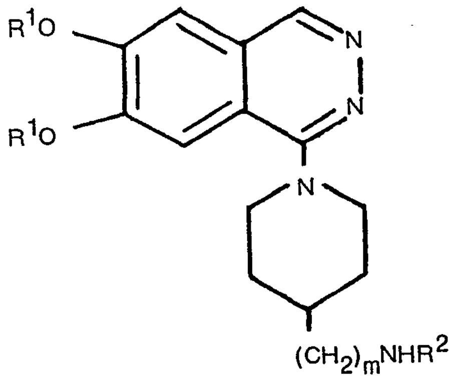

(V)I

with an isocyanate of the formula $R^3NCO$.

In a typical procedure the isocyanate in a suitable solvent (e.g. chloroform) is added dropwise to the phthalazine in a suitable solvent, e.g. dry chloroform, with stirring and cooling. After stirring at room temperature for 1—3 hours the reaction mixture can be concentrated *in vacuo*, triturated with e.g. ethyl acetate and filtered. The resultant solid may be purified by conventional techniques.

The starting materials of the formula (V) are either known compounds or may be prepared by procedures analogous to those of the prior art. The preparation of 6,7-dimethoxy-1-[4-(2-methylaminoethyl)piperidino]phthalazine and 6,7-dimethoxy-1-[4-(methylaminomethyl)piperidino]-phthalazine is for example described in the Preparation 10 of our published British Patent Application No. 2008104A.

Similarly the isocyanates of the formula $R^3NCO$ are either known compounds or may be prepared by procedures analogous to the prior art, e.g. by the reaction of the corresponding amine with phosgene in the presence of triethylamine.

*Route C*

Compounds of the formula (I) in which Y is $-(CH_2)_mN(R^2)COR_3$ or $-(CH_2)_mN(R^2)SO_2R^3$ may be prepared by reacting a compound of the formula (V) as described in Route B with, as appropriate, an acylating agent of the formula $R^3COCl$, $R^3COBr$, $(R^3CO)_2O$, $R^3SO_2Cl$ or $R^3SO_2Br$.

In a typical procedure, the acid or sulfonyl halide in a suitable solvent, e.g. dry chloroform, is added dropwise to a solution of the phthalazine and a base such as triethylamine in a suitable solvent, e.g. dry chloroform, with stirring and cooling. After further stirring at room temperature for 1—3 hours the resulting mixture can be shaken with water, the organic phase dried, and concentrated *in vacuo*. The resulting oil can be chromatographed by a procedure similar to that described in Route A. The product of the chromatographic purification can again be converted to an oxalate salt as described in Route A.

*n*-Butyl lithium may for example be used in place of triethylamine, in which case it is recommended that tetrahydrofuran should be used instead of chloroform.

The following Examples, in which all temperatures are in °C, illustrate the invention:—

## EXAMPLE 1

*Preparation of 6,7-dimethoxy-1-[4-(1,3-dimethyl-3-cyclohexylureidomethyl)piperidino]phthalazine oxalate*

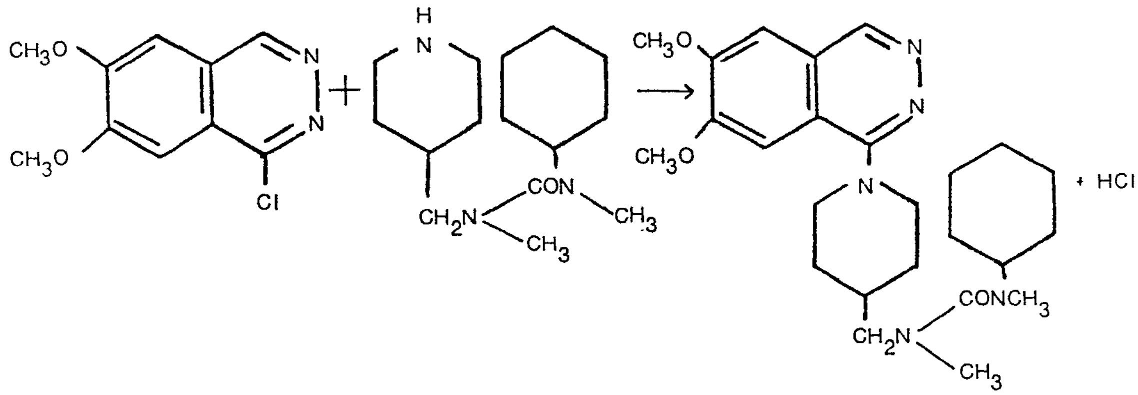

1-Chloro-6,7-dimethoxyphthalazine (1.0 g), 1,3-dimethyl-1-(4-piperidylmethyl)-3-cyclohexylurea (2.8 g), ethanol (25 ml) and triethylamine (5 ml) were heated under reflux for 48 hours. The mixture was then concentrated *in vacuo* to give a black residue which was dissolved in chloroform (30 ml), washed with water (2 x 25 ml) dried and concentrated *in vacuo*. The resultant oil was dissolved in the minimum volume of ethyl acetate and applied to the top of a "Florisil" (Trade Mark)/ethyl acetate column (bed size 16 cm x 1.5 cm). The column was eluted with ethyl acetate/5% ether (100 ml) then neat ethyl acetate. 12 x 30 ml Fractions were collected and the appropriate fractions, which were selected by thin layer chromatography, were combined and concentrated *in vacuo*.

The oxalate salt was precipitated by the addition of excess oxalic acid in isopropanol to a solution of the crude product in the same solvent. Recrystallisation from isopropanol gave 6,7-dimethoxy-1-[4-(1,3-dimethyl-3-cyclohexylureidomethyl)piperidino]phthalazine oxalate hemihydrate (150 mg), m.p. 195—197°.

*Analysis*%:—

| | | | |
|---|---|---|---|
| Found: | C, 58.6; | H, 7.3; | N, 12.3 |
| $C_{25}H_{27}N_5O_3.C_2H_2O_4\frac{1}{2}H_2O$ requires: | C, 58.5; | H, 7.3; | N, 12.6. |

EXAMPLES 2 to 15

The following compounds were prepared similarly to Example 1, starting from 1-chloro-6,7-dimethoxyphthalazine and the appropriate piperidine, and were isolated in the form indicated:

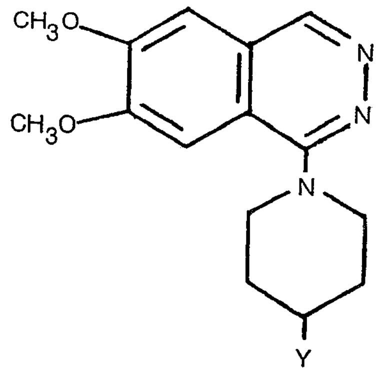

| Example No. | Y | Form Isolated and m.p. (°C) | Analysis % (Theoretical in brackets) C | H | N |
|---|---|---|---|---|---|
| 2 | $-CH_2N(CH_3)CONH.cyclohexyl$ | Hydrochloride hydrate, 210 – 212° | 58.2 (58.1 | 7.1 7.7 | 14.4 14.1) |
| 3 | $-CH_2CH_2N(CH_3)CON(CH_3)cyclopentyl$ | Oxalate hemihydrate, 178 – 179° | 58.6 (58.5 | 7.2 7.3 | 12.1 12.6) |
| 4 | $-CH_2N(CH_3)SO_2.cyclopentyl$ | Oxalate hemihydrate, 203 – 204° | 52.4 (52.6 | 6.5 6.4 | 10.4 10.2) |
| 5 | $-CH_2CH_2N(CH_3)SO_2.cyclohexyl$ | Oxalate, 209 – 210° | 54.9 (55.1 | 6.2 6.8 | 9.4 9.9) |
| 6 | $-CH_2CH_2N(CH_3)SO_2NH.cyclohexyl$ | Hemihydrate 175 – 176° | 57.9 (57.6 | 7.6 7.7 | 13.9 14.0) |
| 7 | $-CH_2CH_2N(CH_3)SO_2NH.cyclopropyl$ | Hydrochloride hydrate, 170 – 171° | 49.7 (50.0 | 6.8 6.8 | 13.7 13.9) |
| 8 | $-CH_2CH_2N(CH_3)\underset{\underset{O}{\|}}{C}O.cyclohexyl$ | Hydrochloride hydrate, 95 – 97° | 58.9 (58.8 | 7.6 7.7 | 10.9 11.0) |
| 9 | $-CH_2N(CH_3)\underset{\underset{O}{\|}}{C}O.cyclohexyl$ | Hydrochloride 226 – 228° | 59.8 (60.1 | 7.4 7.4 | 11.1 11.7) |
| 10 | $-CH_2CH_2NH\underset{\underset{O}{\|}}{C}O.cyclohexyl$ | Hydrochloride hydrate, 110 – 113° | 57.5 (58.0 | 7.1 7.5 | 11.1 11.3) |
| 11 | $-CH_2O\underset{\underset{O}{\|}}{C}NH.cyclohexyl$ | Dihydrochloride, 118 – 119° | 55.2 (55.1 | 6.9 6.8 | 11.4 11.2) |
| 12 | $-CH_2CH_2O\underset{\underset{O}{\|}}{C}NH.cyclohexyl$ | Hydrochloride hydrate, 188° | 57.6 (58.0 | 7.1 7.5 | 11.4 11.3) |

| Example No. | Y | Form Isolated and m.p. (°C) | Analysis % (Theoretical in brackets) C | H | N |
|---|---|---|---|---|---|
| 13 | $-CH_2CH_2N(CH_3)C$.cyclopropyl<br>‖<br>O | Hydrochloride sesquihydrate 116–123° | 57.4<br>(57.2 | 7.6<br>7.4 | 12.1<br>12.1) |
| 14 | $-CH_2OCN(CH_3)$.cyclohexyl<br>‖<br>O | | | | |
| 15 | $-CH_2CH_2N(CH_3)CO$.cyclopentyl | Hydrochloride monohydrate, 170 – 170° | 59.8<br>(59.9 | 7.5<br>7.7 | 11.6<br>11.6) |

EXAMPLE 16

*Preparation of 6,7-dimethoxy-1-[4-{2-(3-cyclohexyl-1-methylureido)ethyl}piperidino]phthalazine*

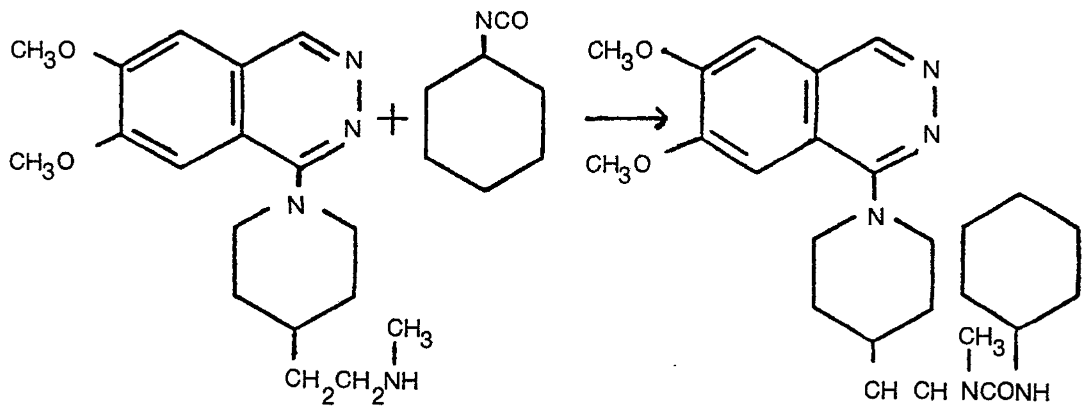

Cyclohexyl isocyanate (0.5 g) in dry chloroform (5 ml) was added dropwise to 6,7-dimethoxy-1-[4-{2-(methylamino)ethyl}piperidino]phthalazine (1.0 g) in dry chloroform (10 ml) with stirring and ice-water cooling. After further stirring at room temperature (20°C) for 1 hour the mixture was concentrated *in vacuo*, triturated with the minimum quantity of ethyl acetate and filtered. The resultant solid was dissolved in methylene chloride (10 ml), filtered, treated with ethyl acetate (20 ml) and gently distilled to remove methylene chloride. The residual ethyl acetate solution was left standing in the refrigerator overnight to precipitate crystalline 6,7-dimethoxy-1-[4-{2-(3-cyclohexyl-1-methylureido)ethyl}piperidino]phthalazine (820 mg), m.p. 204—206°.

*Analysis %:—*

| | | | |
|---|---|---|---|
| Found: | C, 65.6; | H, 8.2; | N, 15.2 |
| $C_{25}H_{37}N_5O_3$ requires: | C, 65.9; | H, 8.2; | N, 15.4. |

## EXAMPLES 17 AND 18

The following compounds were prepared similarly to Example 16, starting from 6,7-dimethoxy-1-[4-{2-(methylamino)ethyl}piperidino]phthalazine or 6,7-dimethoxy-1-[4-(methylaminomethyl)-piperidino]phthalazine and cyclopentyl isocyanate.

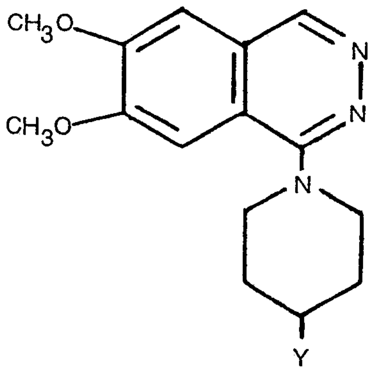

| Example No. | Y | Form Isolated and m.p. (°C) | Analysis % (Theoretical in brackets) C | H | N |
|---|---|---|---|---|---|
| 17 | $-CH_2CH_2N(CH_3)CONH$.cyclopentyl | Hemihydrate 198 – 199° | 64.0 (64.0 | 8.1 8.1 | 15.5 15.5) |
| 18 | $-CH_2N(CH_3)CONH$.cyclopentyl | Free base, 204 – 205° | 64.6 (64.6 | 7.8 7.8 | 16.4 16.4) |

## EXAMPLE 19

*Preparation of 6,7-dimethoxy-1-[4-(2-[N-cyclohexanecarbonyl-N-methylamino]ethyl)piperidino]-phthalazine oxalate*

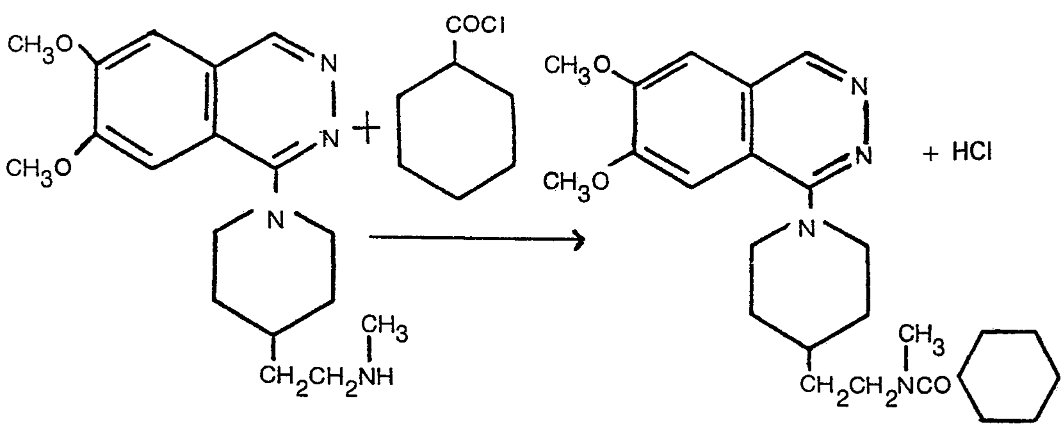

Cyclohexanecarbonyl chloride (0.5 ml) in dry chloroform (5 ml) was added dropwise to a solution of 6,7-dimethoxy-1-{4-[2-(methylamino)ethyl]piperidino}phthalazine (0.5 g) and triethylamine (1 ml) in dry chloroform (10 ml) with stirring and ice-water cooling. After further stirring at room temperature (20°) for 2 hours the mixture was shaken with water (20 ml) and the chloroform phase dried ($Na_2CO_3$) and concentrated *in vacuo*. The resultant oil was dissolved in the minimum volume of ethyl acetate and applied to the top of a "Florisil" (Trade Mark)/ethyl acetate column (bed size 12 x 1.5 cm) followed by elution with ethyl acetate/10% ether. Ten 30 ml fractions were collected and appropriate fractions (identified by thin-layer chromatography) were combined and concentrated *in vacuo*. The residual oil was dissolved in the minimum quantity of isopropanol and converted to the oxalate salt by the addition of an isopropanol solution of oxalic acid to pH 4.0. Recrystallisation of the precipitated solid from isopropanol gave 6,7-dimethoxy-1-{4-(2-[N-cyclohexylcarbonyl-N-methyl-amino]ethyl)piperidino}-phthalazine oxalate hemihydrate (150 mg), m.p. 187—8°.

*Analysis %:—*

| | | | |
|---|---|---|---|
| Found: | C, 60.2; | H, 7.2; | N, 9.9 |
| $C_{25}H_{36}N_4\frac{1}{2}O_3.C_2H_2O_4.H_2O$ requires: | C, 60.1; | H, 7.3; | N, 10.4. |

## EXAMPLES 20—24

The following compounds were prepared similarly to Example 19 starting from 6,7-dimethoxy-1-[4-{2-(methyl-amino)ethyl}piperidino]phthalazine or 6,7-dimethoxy-1-[4-(methylaminoethyl)-piperidino]phthalazine and, either the appropriate acid chloride, or, in the case of Example 24, cyclopropanesulphonyl bromide.

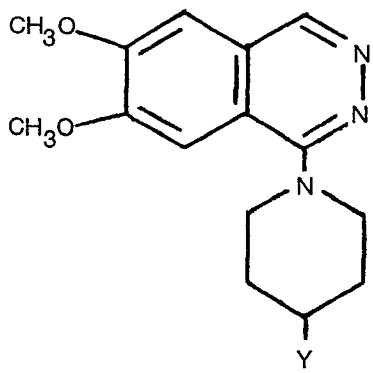

| Example No. | Y | Form Isolated and m.p. (°C) | Analysis % (Theoretical in brackets) C | H | N |
|---|---|---|---|---|---|
| 20 | $-CH_2CH_2N(CH_3CO$.cyclopentyl | Oxalate 1½ hydrate, 153 – 155° | 57.0 (57.4 | 6.7 7.2 | 10.2 10.3) |
| 21 | $-CH_2CH_2N(CH_3)CO$.cyclobutyl | Oxalate hemihydrate, 168 – 171° | 58.1 (58.7 | 6.6 6.9 | 11.2 11.0) |
| 22 | $-CH_2N(CH_3)CO$.cyclopentyl | Dioxalate, 125 – 126° | 54.6 (54.7 | 6.3 6.1 | 9.2 9.5) |
| 23* | $-CH_2N(CH_3).CO$.cyclopropyl | Hemihydrate, 195 – 197° | 64.2 (64.1 | 7.4 7.4 | 14.1 14.2) |
| 24 | $-CH_2CH_2N(CH_3)SO_2$.cyclopropyl | Dihydrochloride, 118° | 49.6 (49.7 | 6.2 6.4 | 11.0 11.0) |

*In this Example *n*-butyllithium was used in place of triethylamine and tetrahydrofuran in place of chloroform.

The following Preparations illustrate the preparation of certain of the starting materials used in the previous Examples (all temperatures are in °C):—

*Preparation 1*

*Preparation of 1-Methyl-1-(4-pyridylmethyl)-3-phenylurea*

4-(Methylaminoethyl)pyridine (5 g), dry chloroform (500 ml) and phenyl isocyanate (5 g) were stirred together at room temperature for 2 hours. The chloroform was then evaporated *in vacuo* and the resultant oil was redissolved in the minimum quantity of chloroform and chromatographed on a "Florisil" (Trade Mark) (100 g) column using chloroform containing gradually increasing quantities of methanol (up to 2.5%) as eluting solvent. Appropriate fractions (identified by t.l.c.) were combined and evaporated *in vacuo* to give 1-methyl-1-(4-pyridylmethyl)-3-phenylurea as an oil (8.0 g).

N.m.r. and mass spectroscopic data were compatible with the required structure:

*Mass spec.:* M⊕ 241.

| | | |
|---|---|---|
| *n.m.r.* ($CDCl_3$) | δ 8.5 (doublet)<br>δ 7.3 (doublet) | Pyridyl ring protons |
| | δ 7 .12 (multiplet) | Phenyl ring protons |
| | δ 4.6 (singlet) | Methylene protons |
| | δ 3.0 (singlet) | N-methyl protons |

*Preparation 2*

*Preparation of 1,3-Dimethyl-1-(4-pyridylmethyl)-3-phenylurea*

Phosgene in toluene (50 ml, 12½% solution) was stirred at 0—10° whilst a mixture of N-methylaniline (5.4 g) and triethylamine (15 ml) in dry chloroform (100 ml) was added slowly dropwise. On completion of the addition the mixture was stirred for 19 hours at room temperature, then cooled to 0—10° whilst a solution of 4-(N-methylaminomethyl)pyridine (3.1 g) in dry chloroform (70 ml) was slowly added. The mixture was stirred at room temperature overnight followed by the cautious addition of 2N hydrochloric acid (20 ml.) After 1 hour, 5N sodium hydroxide (12 ml) was added and the organic phase was separated, washed ($H_2O$, 50 ml), dried ($Na_2CO_3$) and evaporated *in vacuo* to give a dark oil. The oil was dissolved in the minimum quantity of chloroform and chromatographed on a "Florisil" (Trade Mark) column eluted with cloroform. Appropriate fractions were identified by thin layer chromatography, combined and evaporated *in vacuo* to give 1,3-dimethyl-1-(4-pyridylmethyl)-3-phenylurea as a dark oil (3 g). The n.m.r. spectrum was compatible with the required structure.

*Also made by a similar procedure,* starting from phosgene, N-methylaminocyclopentane and 4-[2-(N-methylamino)ethyl]pyridine, was 1,3-dimethyl-1-(2-[4-pyridyl]ethyl)-3-cyclopentylurea (crude oil):

| | | |
|---|---|---|
| *n.m.r.* ($CDCl_3$): | δ 8.49 (doublet)<br>δ 7.10 (doublet) | pyridyl protons; |
| | singlets δ 2.55 and 2.78 (protons of methyl groups); | |
| | triplets at δ~3.4 and δ~3.0 | $—CH_2—CH_2—$ protons |
| | δ 1.4—δ 1.8 (complex signal, cyclopentyl protons) | |

*Preparation 3*

*Preparation of N-methyl-N-(2-[4-pyridyl]ethyl)cyclohexanesulphonamide*

4-(2-[N-methylamino]ethyl)pyridine (5 g) was added dropwise over 15 minutes to a stirred suspension of sodium hydride (1.77 g, 50% dispersion in oil) in dry N,N-dimethylformamide (80 ml) (DMF) containing absolute ethanol (2 drops) under dry nitrogen at room temperature. Upon gentle warming to 50° for 1 hour the coloured anion was generated. After cooling to 0°, cyclohexanesulphonyl chloride (6.71 g) was added dropwise over 45 minutes then the stirred mixture allowed to attain room temperature over 18 hours. The yellow solution was then added to ice-water (200 ml), filtered, extracted with chloroform (25 ml x 4), the organic phase dried ($MgSO_4$) and concentrated *in vacuo*. Residual DMF was distilled from the product at 0.7 mm.Hg and the remaining viscous oil column-chromatographed upon silica (150 g) by elution with chloroform. Evaporation of the primary fractions (200 ml x 3) afforded crystalline N-methyl-N-(2-[4-pyridyl]ethyl)cyclohexane-sulphonamide (8.67 g), m.p. 94—6°.

| | |
|---|---|
| *n.m.r.* ($CDCl_3$) | δ 8.5 (2H, double doublet) |
| | δ 7.15 (2H, double doublet) |
| | δ 3.45 (2H, complex signal) |
| | δ 2.9 (6H, complex signal) |
| | δ 2.2 — 1.0 (10H, envelope of many signals) |

*Also prepared by a similar procedure,* starting from 4-(N-methylaminomethyl)pyridine and cyclopentanesulfonyl chloride, but using triethylamine in chloroform in place of sodium hydride in dimethylformamide, was N-methyl-N-(4-pyridylmethyl)cyclopentanesulphonamide, 2.5 g (crude oil):

| | | |
|---|---|---|
| *n.m.r.* ($CDCl_3$): | δ 8.5 | (2H, double doublet) |
| | δ 7.2 | (2H, double doublet) |
| | δ 4.3 | (2H, singlet) |
| | δ 3.4 | (1H, complex signal) |
| | δ 2.8 | (3H, singlet) |
| | δ 2.3—1.0 | (8H, envelope of many signals) |

*Preparation 4*

*Preparation-4{2-(N-methyl-N-(cyclohexylsulfamoyl)amino]ethyl}pyridine*

4-(2-[N-Methylamino]ethyl)pyridine (15 g) in dry acetonitrile (20 ml) was added dropwise to a cooled (0°C) solution of sulphuryl chloride (15 ml) in dry acetonitrile (50 ml) under a dry, inert atmosphere (nitrogen). After completion of the addition, the suspension was stirred for 1½ hours at 0°C and then for 2 hours at room temperature. The mixture was filtered and the filtrate was evaporated to dryness *in vacuo* to give an oily residue. The residue was redissolved in dry methylene chloride (50 ml) and added dropwise to a cooled solution (0°C) of cyclohexylamine (40 ml) in dry methylene chloride (50 ml). On completion of addition the suspension was boiled under reflux for 3 hours and left to cool overnight. The mixture was filtered and the filtrate was evaporated *in vacuo* to give the product as a crude oil which was chromatographed on a silica column using methylene chloride as eluant. Appropriate fractions were identified by thin layer chromatography, bulked and evaporated to give pure 4-{2-[N-methyl-N-(cyclohexylsulfamoyl)amino]ethyl}pyridine. All impure fractions from the column were bulked and evaporated to give a substantial quantity of impure product (17 g) which was purified by high pressure liquid chromatography on silica gel using methylene chloride:methanol (10:1) as eluant. The pure material obtained from this column was bulked with the pure material obtained from the previous column, total 15.7 g.

*Also prepared by a similar procedure,* from 4-(2-[N-methylamino]ethyl)pyridine, sulfuryl chloride and cyclopropylamine, was 4-{2-[N-methyl-N-(cyclopropylsulfamoyl)amino]ethyl}pyridine, m.p. 99—101°C.

*n.m.r.*: $CDCl_3$:

δ 8.48, 2H, double doublet; δ 7.15, 2H, double doublet;
δ 3.4, 2H, multiplet; δ 2.9, 2H, multiplet; δ 2.8, 3H, singlet;
δ 2.4, 1H, quintet; δ 0.62, 4H, doublet.

*Preparation 5*

*Synthesis of 4-(2-{N-cyclopentanecarbonyl-N-methylamino}ethyl-pyridine dioxalate*

Cyclopentanecarboxylic acid (11.4 g) in dry tetrahydrofuran (15 ml) was added dropwise to a stirred suspension of 1,1′-carbonyldiimidazole (17.8 g) in tetrahydrofuran (95 ml) at room temperature. Stirring was continued at room temperature until evolution of carbon dioxide gas ceased (1 hour). 4-(2-{N-methylamino}ethyl-pyridine (15 g) in tetrahydrofuran (20 ml) was then added dropwise, after which the reaction mixture was allowed to stand at room temperature for 60 hours. After removal of the solvent by distillation *in vacuo* the residue was suspended in water (200 ml) and extracted with chloroform (2 x 200 ml). The combined chloroform extracts were dried ($MgSO_4$) and evaporated *in vacuo* to give a clear oil which was redissolved in the minimum volume of ethyl acetate and applied to a medium pressure chromatography column (prepared from Keiselgel (Trade Mark) H Type 60, 120 g) (product of Merck GmBH). Elution was achieved with ethyl acetate containing increasing proportions of methanol (0→20%) and 8 fractions of 150 ml each were collected. Thin layer chromatography indicated that product was present in fractions 1 to 7 together with only a trace of impurity. Fractions 1 to 7 were combined, and then evaporation of the solvent gave a clear oil (19.9 g). A portion of this oil was dissolved in the minimum quantity of ethyl acetate and acidified to pH 3 (as measured by moist pH paper) with a saturated solution of oxalic acid in ethyl acetate. The precipitated product recrystallised from acetonitrile to give white crystals of the title compound, m.p. 108—110°.

*Analysis %:—*

| | | | |
|---|---|---|---|
| Found: | C, 52.5; | H, 5.9; | N, 6.9. |
| $C_{14}H_{20}N_2O.2(C_2H_2O_4)$ requires: | C, 52.4; | H, 5.9; | N, 6.8. |

*Also prepared similarly to the above,* starting from cyclopropanecarboxylic acid, 1,1′-carbonyldiimidazole and 4-(2-[N-methylamino]ethyl)pyridine, was 4-(2-[N-cyclopropanecarbonyl-N-methylamino]ethyl)pyridine, characterised as the dioxolate m.p. 117—119°.

*Analysis %:—*

| | | | |
|---|---|---|---|
| Found: | C, 50.0; | H, 5.3; | N, 7.5. |
| $C_{12}H_{16}N_2O.2(C_2H_2O_4)$: | C, 50.0; | H, 5.3; | N, 7.3. |

*Preparation 6*

*4-[N-cyclohexylcarbamoyloxymethyl]pyridine*

1,1′-Carbonyldiimidazole (8.1 g) in dry tetrahydrofuran (50 ml) was stirred at room temperature under a dry nitrogen atmosphere whilst 4-hydroxymethylpyridine (5.5 g) in tetrahydrofuran (THF) (25 ml) was added dropwise. The mixture was stirred at room temperature overnight and was then treated dropwise with cyclohexylamine (4.6 g) in THF (25 ml), after which it was stirred for 4 hours and then boiled under reflux for 2 hours. The solvent was removed by evaporation *in vacuo* and the residual yellow solid was recrystallised from a mixture of ethyl acetate and methanol to give 4-[N-cyclohexylcarbamoyloxymethyl]pyridine (6 g), m.p. 126—7°.

*Analysis %:—*

| | | | |
|---|---|---|---|
| Found: | C, 66.7; | H, 7.8; | N, 12.1. |
| $C_{13}H_{18}N_2O_2$ requires: | C, 66.6; | H, 7.7; | N, 12.0. |

*Also prepared similarly to the above,* starting from 1,1′-carbonyldiimidazole, 1-benzyl-4-(2-hydroxyethyl)piperidine and cyclohexylamine, was: 1-benzyl-4-[2-(N-cyclohexylcarbamoyloxy)ethyl]-piperidine, as a tan coloured oil.

*n.m.r. data: $CDCl_3$:*
$\delta$ 7.3 (5H) multiplet, $\delta$ 4.18, 3H, multiplet; $\delta$ 3.5, 2H, singlet;
$\delta$ 2.9, 2H, ill-defined doublet; $\delta$ 1.5, 20H complex mound.

*Also prepared similarly to the above,* starting from 1,1′-carbonyldiimidazole, 4-hydroxymethylpyridine and N-methylcyclohexylamine, was 4-[N-methyl-N-cyclohexylcarbamoyloxy-methylpyridine.

*n.m.r.* ($CDCl_3$):
$\delta$ 8.6 (2H, dd), $\delta$ 7.2 (2H, dd), $\delta$ 5.2 (2H, s), $\delta$ 3.85 (1H, brd), $\delta$ 2.85 (3H, S), and $\delta$ 2.0—1.1 (10H, brd, m).

*Preparation 7*

*4-[N-methyl-N-cyclohexyloxycarbonyl)aminomethyl]pyridine oxalate*

1,1′-Carbonyldiimidazole (8.1 g) in dry tetrahydrofuran (THF) (50 ml) was stirred at room temperature under a dry nitrogen atmosphere whilst a solution of cyclohexanol (5.0 g) in THF (25 ml) was added dropwise. The mixture was stirred at room temperature overnight and was then treated dropwise with a solution of 4-(N-methylaminomethyl)pyridine (6.1 g) in THF (25 ml) after which it was boiled under reflux for 8 hours. The mixture was left at room temperature for 5 days and was then evaporated to dryness *in vacuo* to give the product as a mobile yellow oil (8 g). A portion of this was converted to the title oxalate salt, m.p. 108°.

*Analysis %:—*

| | | | |
|---|---|---|---|
| Found: | C, 57.3; | H, 6.8; | N, 7.9 |
| $C_{14}H_{20}N_2O_2.C_2H_2O_4$ requires: | C, 56.8; | H, 6.6; | N, 8.3. |

*Also prepared by a similar procedure,* but starting from 4-(2-[N-methylamino]ethyl)pyridine, 1,1′-carbonyldiimidazole and cyclohexanol, was 4-{2-[(N-methyl-N-cyclohexyloxycarbonyl)amino]-ethyl}pyridine oxalate, m.p. 109°.

*n.m.r.* ($CDCl_3$ — free base):
$\delta$ 8.48, 2H, double doublet; $\delta$ 7.12, 2H, double doublet;
$\delta$ 3.5, 2H, multiplet; $\delta$ 2.85, 5H, singlet over multiplet;
$\delta$ 1.5, 10H complex mound.

*Also prepared by a similar procedure,* but starting from 4-(2-aminoethyl)pyridine, 1,1′-carbonyldiimidazole and cyclohexanol, was 4-(2-[N-cyclohexyloxycarbonylamino]ethyl)pyridine oxalate hemihydrate, m.p. 110°.

*Analysis %:—*

| | | | |
|---|---|---|---|
| Found: | C, 55.4; | H, 6.3; | N, 8.5 |
| $C_{14}H_{20}N_2O_2.C_2H_2O_4.\frac{1}{2}H_2O$ requires: | C, 55.3; | H, 6.7; | N, 8.1. |

*Preparation 8*

*Preparation of Cyclopropanesulphonyl bromide*

Cyclopropyllithium was prepared *in situ* by the dropwise addition of cyclopropylbromide (9.97 g) in dry ether (20 ml) to a stirred suspension of lithium wire (1.14 g) in ether (20 ml) at 0° under dry nitrogen. Attrition with glass chips and a crystal of iodine initiated the reaction, which was complete after 2 hours.

Sulphur dioxide gas was condensed into a flask under $N_2$ and held at —60°, to which was added dropwise *via* a catheter with stirring the cold cyclopropyllithium solution; lithium bromide by-product was also carried over at this stage. Upon warming to 0° the solvent was evaporated from the cream coloured suspension *in vacuo.* Dry dichloromethane (30 ml) was then added with stirring at 0° followed by sulphuryl chloride (7 ml) dropwise over 10 minutes. The mixture was stirred for 1 hour at 0° then warmed to room temperature overnight.

After adding further dichloromethane (20 ml) the solids were filtered off and the solvent removed *in vacuo.* Upon brief cooling the residual oil deposited a small crystalline mass (unidentified) which was filtered off and the filtrate fractionally distilled *in vacuo,* collecting the fraction with b.p. (0.3 mmHg) 49—54°, yield of cyclopropanesulphonyl bromide 2.8 g.

$^m/e$ no $M^+$, but $^m/e$ 105 ≡ ▷—$SO_2^+$
*i.r.* (Thin film) 3060, 1360, 1160, 870 and 675 $cm^{-1}$.
*n.m.r.* ($CDCl_3$) $\delta$ 3.50 (1H, multiplet) and 1.50 (4H, multiplet) p.p.m. Elemental analysis (Found Cl — 1.25%, Br — 40.1%) indicated the product was 93% cyclopropanesulphonylbromide and 7% chloride.

### Preparation 9

### *Preparation of 1-Benzyl-4-(2-hydroxyethyl)piperidine*

To a solution of 4-(2-hydroxyethyl)piperidine (20 g) in absolute ethanol (200 ml) at room temperature was added benzaldehyde (16.5 g), and the mixture was stirred overnight. A solution of sodium borohydride (5.6 g) in absolute ethanol (200 ml) was then added dropwise with stirring and the mixture cooled in an ice-bath. After the reaction was complete (as shown by t.l.c.), methanol (10 ml) was added to destroy any excess sodium borohydride and the mixture evaporated to dryness *in vacuo*. The residue was dissolved in the minimum of absolute ethanol and passed down a short (10 cm x 6 cm dia.) dry silica column and eluted with absolute ethanol to remove inorganic material. Upon evaporation of the solvent *in vacuo* the colourless oily product (33 g) was obtained, and was used directly without further purification.

*Data* Crude yield (33 g)

*N.M.R.* ($d^6$DMSO) $\delta$ 7.25 (5H, s); 4.45 (1H, brd, exch.); 3.4 (2H, brd, s); 2.8 (2H, brd, m); and 2.2 — 1.0 (11H, brd, m).

### Preparation 10

### *Preparation of 1-Methyl-1-(4-piperidylmethyl)-3-cyclohexylurea*

1-Methyl-1-(4-pyridylmethyl)-3-phenylurea (7.0 g) in glacial acetic acid (50 ml) was hydrogenated over platinum oxide at 60°/60 p.s.i. until uptake of hydrogen ceased. The catalyst was removed by filtration and the filtrate evaporated *in vacuo*. The resultant oil was basified with 5N NaOH (to pH 12) and extracted with chloroform (2 x 50 ml). The combined extracts were dried ($MgSO_4$) and evaporated to give 1-methyl-1-(4-piperidylmethyl)-3-cyclohexylurea as an oil (4.0 g). The n.m.r. and mass spectra were compatible with the required structure.

The following compounds were prepared in a similar manner from the corresponding pyridine derivatives, or, in the case of (l), from the 1-benzylpiperidine.

(a) 1,3-Dimethyl-1-(4-piperidylmethyl)-3-cyclohexylurea (crude oil) (structure confirmed by n.m.r.).

(b) 1,3-Dimethyl-1-(2-[4-piperidyl]ethyl)-3-cylopentylurea (crude oil):

*n.m.r.* ($CDCl_3$): singlets at $\delta$ 2.68 and 2.80 p.p.m. (1,3 dimethyl protons)

| | | |
|---|---|---|
| (on acetate salt form) | complex signals at $\delta$ 1.3—1.8 p.p.m. | (shielded cyclic alkane protons) |
| | complex signals at $\delta$ 2.5—2.9<br>3.0—3.5 | deshielded cyclic alkane and straight chain protons adjacent to nitrogens |

*i.r.* $\nu$ C = 0: 1625 $cm^{-1}$

(c) N-methyl-N-(4-piperidylmethyl)cyclopentanesulphonamide:

*n.m.r. $CDCl_3$*): $\delta$ 3.6 — 2.4 (11H, envelope signals including those of the —$NCH_3$ protons at $\delta$ 2.9 — singlet)
$\delta$ 2.4 — 1.0 (13H, envelope of signals)

(d) N-methyl-N-(2-[4-piperidyl]ethyl)cyclohexanesulphonamide:

*n.m.r.* ($CDCl_3$): $\delta$ 3.85 (1H, $D_2O$ exchangeable)
$\delta$ 3.4 — 2.4 (10H, complex signal)
$\delta$ 2.4 — 0.9 (17H, envelope of many signals).

(e) 4-{2-[N-methyl-N-(cyclohexylsulfamoyl)amino]ethyl}piperidine
m.p. 105 — 106°.

*n.m.r.* ($CDCl_3$): $\delta$ 3.1, 6H multiplet; $\delta$ 2.75, 3H, singlet;
$\delta$ 2.57, 2H multiplet; $\delta$ 1.3, 18H complex mound.

(f) 4-{2-[N-methyl-N-(cyclopropylsulfamoyl)amino]ethyl}piperidine acetate quarter hydrate, m.p. 127—129°.

*Analysis %:—*

| | | | |
|---|---|---|---|
| Found: | C, 47.7; | H, 8.3; | N, 12.8 |
| $C_{11}H_{23}N_3O_2S.C_2H_4O_2\frac{1}{4}H_2O$ requires: | C, 47.9; | H, 8.5; | N, 12.9. |

(g) 4-(2-{N-cyclopentanecarbonyl-N-methylamino}ethyl)piperidine oxalate, m.p. 148—150°.

*Analysis %:—*

| | | | |
|---|---|---|---|
| Found: | C, 58.2; | H, 8.9; | N, 8.5 |
| Calculated for $C_{14}H_{26}N_2O.C_2H_2O_4$: | C, 58.5; | H, 8.6; | N, 8.5. |

(h) 4-{(N-methyl-N-cyclohexyloxycarbonyl)aminomethyl}piperidine oxalate hemihydrate, m.p. 125°.

*Analysis %:—*

| | | | |
|---|---|---|---|
| Found: | C, 54.1; | H, 8.1; | N, 8.1 |
| $C_{14}H_{26}N_2O_2.C_2H_2O_4.\frac{1}{2}H_2O$ requires: | C, 54.4; | H, 8.3; | N, 7.9. |

(i) 4-[2-{(N-methyl-N-cyclohexyloxycarbonyl)amino}ethyl]piperidine oxalate, m.p. 137°.

*Analysis %:—*

| | | | |
|---|---|---|---|
| Found: | C, 56.4; | H, 8.93; | N, 7.7 |
| $C_{15}H_{28}N_2O_2.C_2H_2O_4$ requires: | C, 57.0; | H, 8.4; | N, 7.8. |

(j) 4-[2-(cyclohexyloxycarbonylamino)ethyl]piperidine oxalate monohydrate, m.p. 156°.

*Analysis %:—*

| | | | |
|---|---|---|---|
| Found: | C, 53.1; | H, 7.9; | N, 7.7 |
| $C_{14}H_{26}N_2O_2.C_2H_21O_4.H_2O$ requires: | C, 53.0; | H, 8.3; | N, 7.7. |

(k) 4-[(N-cyclohexyl)carbamoyloxymethyl]piperidine hemioxalate hemihydrate, m.p. 242°.

*Analysis %:—*

| | | | |
|---|---|---|---|
| Found: | C, 56.9; | H, 9.3; | N, 9.4. |
| $C_{13}H_{24}N_2O_2.\frac{1}{2}(C_2H_2O_4).\frac{1}{2}H_2O$ requires: | C, 57.1; | H, 8.9; | N, 9.5. |

(l) 4-(2-[N-cyclohexylcarbamoyloxy]ethyl)piperidine, an oil.
*n.m.r.* ($CDCl_3$): $\delta$ 4.1, 3H multiplet; $\delta$ 2.3 — 2.8, 6H, complex signal;
$\delta$ 1.4, 18H, complex mound.

This was prepared by a slight modification of the above procedure using ethanol as the solvent and the catalyst as 5% Pd/C.

(m) 4-(2-[N-methyl-N-cyclopropanecarbonylamino]ethyl)piperidine, used directly in Example 13; *and*

(n) 4-(N-methyl-N-cyclohexylcarbamoyloxymethyl)piperidine, used directly in Example 14.

**Claims for the contracting states: BE, CH, DE, FR, GB, IT, LU, NL, SE**

1. A compound of the formula:

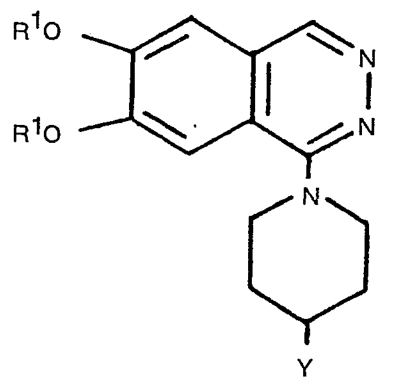

(I)

wherein $R^2$ is a $C_1$—$C_4$ group, and Y is a group of the formula: —$(CH_2)_m$—Z, wherein m is 1 or 2, and Z is a group selected from:

—$OCONR^3R^4$,
—$N(R^2)COR^3$,
—$N(R^2)SO_2R^3$,
—$N(R^2)CONR^3R^4$,
—$N(R^2)SO_2NR^3R^4$,
and —$N(R^2)COOR^3$,

wherein $R^2$ and $R^4$ are each independently hydrogen or $C_1$—$C_4$ alkyl, and $R^3$ is $C_3$—$C_7$ cycloalkyl; or a pharmaceutically acceptable acid addition salt threof.

2. A compound as claimed in claim 1 wherein $R^1$ is methyl, $R^2$ and $R^4$ are each independently hydrogen or methyl, and $R^3$ is $C_3$—$C_6$ cycloalkyl.

3. A compound as claimed in claim 1 wherein $R^1$ is methyl and Z is selected from:

(a) —$OCON(R^4)$ (cyclohexyl) wherein $R^4$ is H or $CH_3$;
(b) —$N(CH_3)COR^3$ wherein $R^3$ is $C_3$—$C_6$ cycloalkyl;
(c) —$N(CH_3)SO_2R^3$ wherein R is cyclopropyl, cyclopentyl or cyclohexyl;

(d) —$N(CH_3)CONR^3N^4$ wherein $R^3$ is cyclopentyl or cyclohexyl and $R^4$ is H or $CH_3$;
(e) —$N(CH_3)SO_2NHR^3$ wherein $R^3$ is cyclopropyl or cyclohexyl; *and*
(f) —$N(R^2)COO$.cyclohexyl wherein $R^2$ is H or $CH_3$.

4. A compound as claimed in claim 1 wherein $R^1$ is $CH_3$ and Y is selected from —$CH_2CH_2N(CH_3)CON(CH_3)$.cyclopentyl, —$CH_2CH_2N(CH_3)CO$.cyclopentyl and —$CH_2CH_2N(CH_3)SO_2NH$.cyclohexyl.

5. A process for preparing a compound of the formula (I) as claimed in claim 1 which comprises reacting a phthalazine of the formula:—

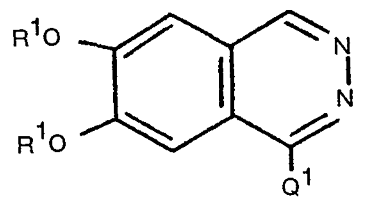

(III)

wherein $R^2$ is as defined in claim 1, and $Q^1$ is a facile leaving group, with an amine of the formula:

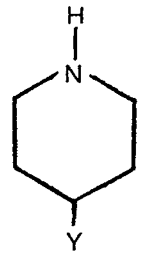

(IV)

wherein Y is as defined in claim 1.

6. A process for preparing a compound of the formula (I) as claimed in claim 1 wherein Y is —$(CH_2)_mN(R^2)CONHR^3$ wherein m, $R^2$ and $R^3$ are as defined in claim 1, which comprises reacting a phthalazine of the formula:

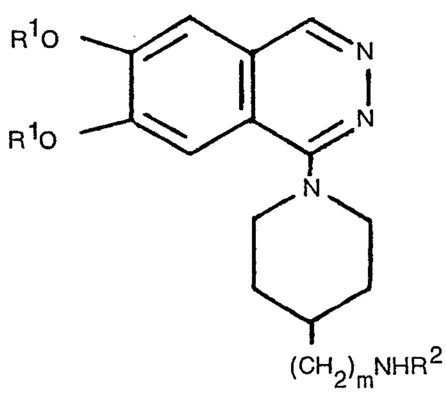

(V)

wherein $R^1$, m and $R^2$ are as defined in claim 1, with an isocyanate of the formula $R^3NCO$ wherein $R^3$ is as defined in claim 1.

7. A process for preparing a compound of the formula (I) as claimed inn claim 1 wherein Y is —$(CH_2)_mN(R^2)COR^3$ or —$(CH_2)_mN(R^2)SO_2R^3$ wherein m, $R^2$ and $R^3$ are as defined in claim 1, which comprises reacting a compound of the formula (V) as defined in claim 6 with, as appropriate, an acylating agent of the formula $R^3COCl$, $R^3COBr$, $(R^3CO)_2O$, $R^3SO_2Cl$ or $R^3SO_2Br$, wherein $R^3$ is as defined in claim 1.

8. A compound of the formula (I) as claimed in any one of claims 1 to 4, or a pharmaceutically acceptable acid addition salt thereof, for use in treating heart failure in a human being.

9. A pharmaceutical composition comprising a compound of the formula (I) as claimed in any one of claims 1 to 4 or a pharmaceutically acceptable acid addition salt thereof, together with a pharmaceutically acceptable diluent or carrier.

**Patentansprüche für die Vertragsstaaten BE, CH, DE, FR, GB, IT, LU, NL, SE**

1. Verbindung der Formel

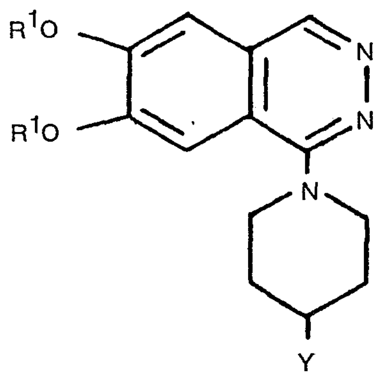

(I),

worin $R^1$ eine $C_1$—$C_4$-Alkylgruppe und Y eine Gruppe der Formel —$(CH_2)_m$—Z, worin m 1 oder 2 ist, und Z eine Gruppe, ausgewählt unter

—$OCONR^3R^4$,
—$N(R^2)COR^3$,
—$N(R^2)SO_2R^3$,
—$N(R^2)CONR^3R^4$,
—$N(R^2)SO_2NR^3R^4$,
und —$N(R^2)COOR^3$,

worin $R^2$ und $R^4$ jeweils unabhängig voneinander Wasserstoff oder $C_1$—$C_4$-Alkyl sind und $R^3$ $C_3$—$C_7$-Cycloalkyl ist, oder ein pharmazeutisch annehmbares Säureadditionssalz hiervon.

2. Verbindung nach Anspruch 1, worin $R^1$ Methyl ist, $R^2$ und $R^4$ unabhängig voneinander jeweils Wasserstoff oder Methyl sind und $R^3$ $C_3$—$C_6$-Cycloalkyl ist.

3. Verbindung nach Anspruch 1, worin $R^1$ Methyl und Z unter
(a) —$O.CON(R^4)$ (Cyclohexyl), worin $R^4$ H oder $CH_3$ ist,
(b) —$N(CH_3)COR^3$, worin $R^3$ $C_3$—$C_6$-Cycloalkyl ist,
(c) —$N(CH_3)SO_2R^3$, worin $R^3$ Cyclopropyl, Cyclopentyl oder Cyclohexyl ist,
(d) —$N(CH_3)CONR^3R^4$, worin $R^3$ Cyclopentyl oder Cyclohexyl und $R^4$ H oder $CH_3$ ist,
(e) —$N(CH_3)SO_2NH.R^3$, worin $R^3$ Cyclopropyl oder Cyclohexyl ist, und
(f) —$N(R^2)COO$-Cyclohexyl, worin $R^2$ H oder $CH_3$ ist.

4. Verbindung nach Anspruch 1, worin $R^1$ $CH_3$ und Y unter —$CH_2CH_2N(CH_3)CON(CH_3)$-Cyclopentyl, —$CH_2CH_2N(CH_3)CO$-Cyclopentyl und —$CH_2CH_2N(CH_3)SO_2NH$-Cyclohexyl ausgewählt ist.

5. Verfahren zur Herstellung einer Verbindung der Formel (I) gemäß Anspruch 1, bei dem ein Phthalazin der Formel

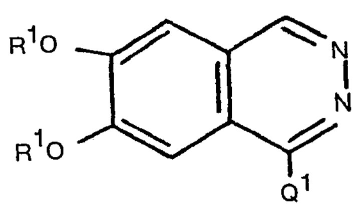

(III),

worin $R^1$ wie in Anspruch 1 definiert und $Q^1$ eine leicht austretende Gruppe, mit einem Amin der Formel

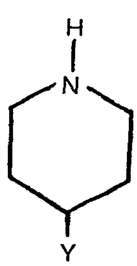

(IV),

worin Y wie in Anspruch 1 definiert ist, umgesetzt wird.

6. Verfahren zur Herstellung einer Verbindung der Formel (I) gemäß Anspruch 1, worin Y —$(CH_2)_mN(R^2)CONHR^3$ ist, worin m, $R^2$ und $R^3$ wie in Anspruch 1 definiert sind, bei dem ein Phthalazin der Formel

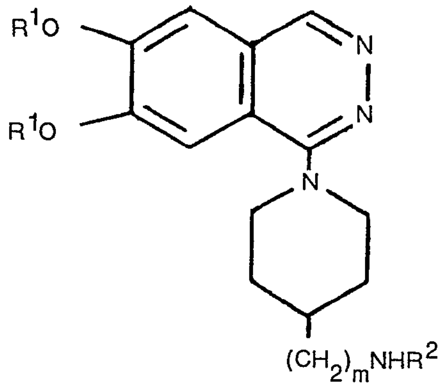

(V),

worin $R^1$, m und $R^2$ wie in Anspruch 1 definiert sind, mit einem Isocyanat der Formel $R^3NCO$, worin $R^3$ wie in Anspruch 1 definiert ist, umgesetzt wird.

7. Verfahren zur Herstellung einer Verbindung der Formel (I) gemäß Anspruch 1, worin Y $-(CH_2)_mN(R^2)COR^3$ oder $-(CH_2)_mN-(R^2)SO_2R^3$ ist, worin m, $R^2$ und $R^3$ wie in Anspruch 1 definiert sind, bei dem eine Verbindung der Formel (V), wie in Anspruch 6 definiert, mit, nach Eignung, einem Acylierungsmittel der Formel $R^3COCl$, $R^3COBr$, $(R^3CO)_2O$, $R^3SO_2Cl$ oder $R^3SO_2Br$, worin $R^3$ wie in Anspruch 1 definiert ist, umgesetzt wird.

8. Verbindung der Formel (I) gemäß einem der Ansprüche 1 bis 4 oder ein pharmazeutisch annehmbares Säureadditionssalz hiervon zur Verwendung bei der Behandlung von Herzversagen beim Menschen.

9. Pharmazeutisches Mittel mit einer Verbindung der Formel (I) gemäß einem der Ansprüche 1 bis 4 oder ein pharmazeutisch annehmbares Säureadditionssalz hiervon, zusammen mit einem pharmazeutisch annehmbaren Verdünnungsmittel oder Träger.

**Revendications pour les Etats contractants: BE, CH, DE, FR, GB, IT, LU, NL, SE**

1. Composé de formule:

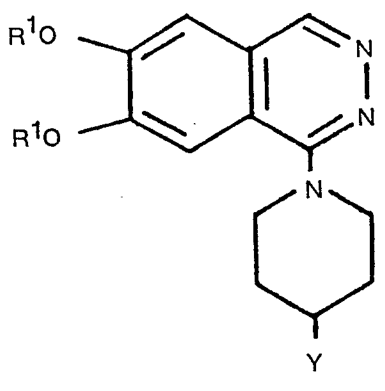

(I)

dans laquelle $R^1$ est un groupe alkyle en $C_1$ à $C_4$ et Y est un groupe de formule: $-(CH_2)_m-Z$, où m est égal à 1 ou 2, et Z est un groupe choisi entre:

$-OCONR^3R^4$,
$-N(R^2)COR^3$,
$-N(R^2)SO_2R^3$,
$-N(R^2)CONR^3R^4$,
$-N(R^2)SO_2NR^3R^4$,
et $-N(R^2)COOR^3$,

où $R^2$ et $R^4$ représentent chacun, indépendamment, l'hydrogène ou un radical alkyle en $C_1$ à $C_4$ est un radical cycloalkyle en $C_3$ à $C_7$; ou un sel d'addition d'acide pharmaceutiquement acceptable de ce composé.

2. Composé suivant la revendication 1, dans lequel $R^1$ est le groupe méthyle, $R^2$ et $R^4$ représentent chacun, indépendamment, de l'hydrogène ou le groupe méthyle et $R^3$ est un groupe cycloalkyle en $C_3$ à $C_6$.

3. Composé suivant la revendication 1, dans lequel $R^1$ est le groupe méthyle et Z est choisi entre:

(a) un groupe de formule $-OCON(R^4)$ (cyclohexyle) dans lequel $R^4$ représente H ou $CH_3$;
(b) un groupe de formule $-N(CH_3)COR^3$ dans laquelle $R^3$ est un radical cycloalkyle en $C_3$ à $C_6$;
(c) un groupe de formule $-N(CH_3)SO_2R^3$ dans laquelle $R^3$ est un reste cyclopropyle, cyclopentyle ou cyclohexyle;

(d) un groupe de formule $—N(CH_3)CONR^3R^4$ dans laquelle $R^3$ est un reste cyclopentyle ou cyclohexyle et $R^4$ représente H ou $CH_3$;

(e) un groupe de formule $—N(CH_3)SO_2NHR^3$ dans laquelle $R^3$ est un reste cyclopropyle ou cyclohexyle; et

(f) un groupe de formule $—N(R^2)COO$.cyclohexyle dans laquelle $R^2$ représente H ou $CH_3$.

4. Composé suivant la revendication 1, dans lequel $R^1$ représente $CH_3$ et Y est choisi entre les groupes $—CH_2CH_2N(CH_3)CON(CH_3)$.cyclopentyle, $—CH_2CH_2N(CH_3)CO$.cyclopentyle et $—CH_2CH_2N(CH_3)SO_2NH$.cyclohexyle.

5. Procédé de préparation d'un composé de formule (I) suivant la revendication 1, caractérisé en ce qu'il consiste à faire réagir une phtalazine de formule:

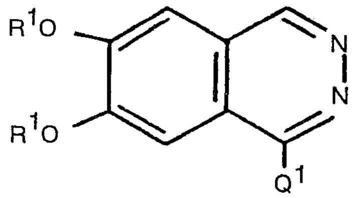

(III)

dans laquelle $R^1$ a la définition donnée dans la revendication 1 et $Q^1$ est un groupe aisément partant, avec une amine de formule:

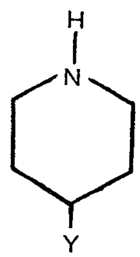

(IV)

dans laquelle Y a la définition donnée dans la revendication 1.

6. Procédé de préparation d'un composé de formule (I) suivant la revendication 1, dans laquelle Y est un groupe $—(CH_2)_mN(R^2)CONHR^3$ dans lequel m, $R^2$ et $R^3$ ont les définitions données dans la revendication 1, caractérisé en ce qu'il consiste à faire réagir une phtalazine de formule:

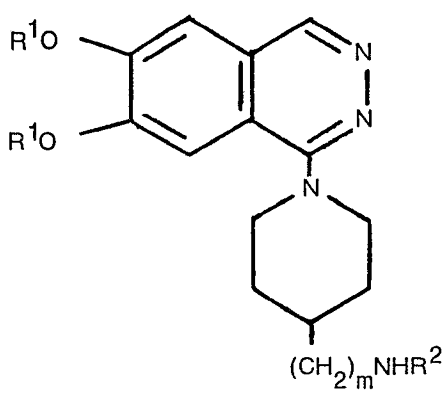

(V)

dans laquelle $R^1$, m et $R^2$ ont les définitions données dans la revendication 1, avec un isocyanate de formule $R^3NCO$ dans laquelle $R^3$ a la définition donnée dans la revendication 1.

7. Procédé de préparation d'un composé de formule (I) suivant la revendication 1, dans laquelle Y est un groupe $—(CH_2)_mN(R^2)COR^3$ ou $—(CH_2)_mN(R^2)SO_2R^3$, où m, $R^2$ et $R^3$ ont les définitions données dans la revendication 1, caractérisé en ce qu'il consiste à faire réagir un composé de formule (V) comme défini dans la revendication 6 avec, selon le cas, un agent acylant de formule $R^3COCl$, $R^3COBr$, $(R^3CO)_2O$, $R^3SO_2Cl$ ou $R^3SO_2Br$, où $R^3$ a la définition donnée dans la revendication 1.

8. Un composé de formule (I) suivant l'une quelconque des revendications 1 à 4, ou un sel d'addition d'acide pharmaceutiquement acceptable de ce composé, destiné à être utilisé dans le traitement de l'insuffisance cardiaque chez un être humain.

9. Composition pharmaceutique, caractérisée en ce qu'elle comprend un composé de formule (I) suivant l'une quelconque des revendications 1 à 4 ou un sel d'addition d'acide pharmaceutiquement acceptable de ce composé, conjointement avec un diluant ou support acceptable du point de vue pharmaceutique.

**Claims for the contracting state: AT**

1. A process for preparing a phthalazine of the formula:

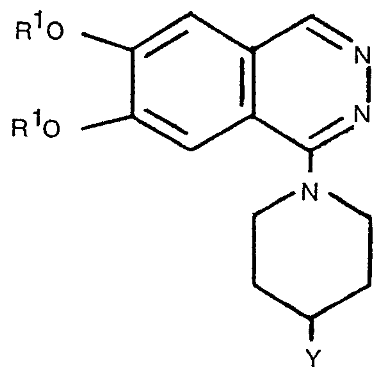

(I)

wherein $R^1$ is a $C_1—C_4$ alkyl group, and Y is a group of the formula: $—(CH_2)_m—Z$, wherein m is 1 or 2, and Z is a group selected from:

—$OCONR^3R^4$,
—$N(R^2)COR^3$,
—$N(R^2)SO_2R^3$,
—$N(R^2)CONR^3R^4$,
—$N(R^2)SO_2NR^3R^4$,
and —$N(R^2)COOR^3$,

wherein $R^2$ and $R^4$ are each independently hydrogen or $C_1—C_4$ alkyl, and $R^3$ is $C_3—C_7$ cycloalkyl; or a pharmaceutically acceptable acid addition salt thereof; characterised by reacting a phthalazine of the formula:

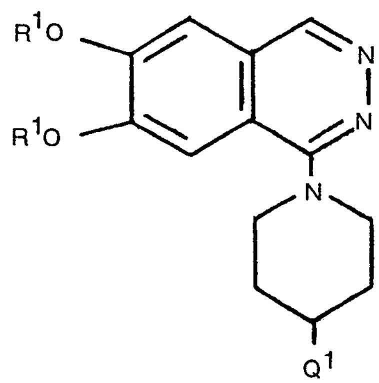

(III)

wherein R1 is as defined above and $Q^1$ is a facile leaving group such as chloro, bromo, iodo, lower alkoxy or (lower alkyl)thio, with an amine of the formula:

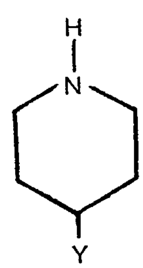

(IV)

wherein Y is as defined above; followed by, optionally, reacting the thus produced product of the formula (I) with a non-toxic acid to form a pharmaceutically acceptable acid addition salt.

2. A process for preparing a phthalazine of the formula:

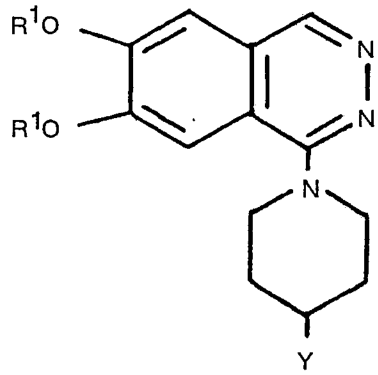

(I)

wherein $R^1$ is $C_1$—$C_4$ alkyl, and Y is —$(CH_2)_m N(R^2)CONHR^3$, —$(CH_2)_m N(R^2)COR^3$ or —$(CH_2)_m N(R^2)SO_2R^3$, wherein m is 1 or 2, $R^2$ is $C_1$—$C_4$ alkyl and $R^3$—$C_7$ cycloalkyl characterised by reacting a phthalazine of the formula:

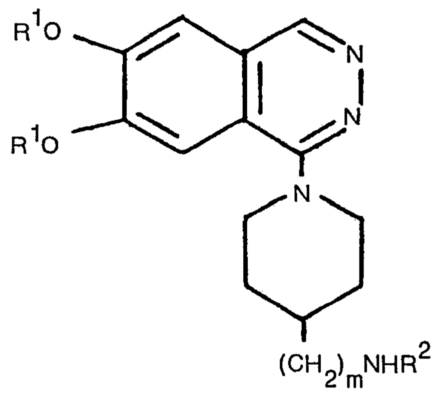

(V)

wherein m, $R^1$ and $R^2$ are as defined above, with, as appropriate, a compound of the formula $R^3X$ or $(R^3CO)_2O$ wherein X is, as appropriate, —NCO, —COCl, —COBr, —$SO_2Cl$ or —$SO_2Br$; followed by, optionally, reacting the thus-produced product of the formula (I) with a non-toxic acid to form a pharmaceutically acceptable acid addition salt.

3. A process as claimed in claim 1 or 2 wherein $R^1$ is $CH_3$, $R^2$ is H or $CH_3$ and $R^3$ is $C_3$—$C_6$ cycloalkyl.

4. A process as claimed in claim 1 wherein $R^1$ is $CH_3$ and Y is selected from —$CH_2CH_2N(CH_3)CON(CH_3)$.cyclopentyl, —$CH_2CH_2N(CH_3)CO$.cyclopentyl and —$CH_2CH_2N(CH_3)SO_2NH$.cyclohexyl.

**Patentansprüche für der Vertragsstaat: AT**

1. Verfahren zur Herstellung eines Phthalazins der Formel

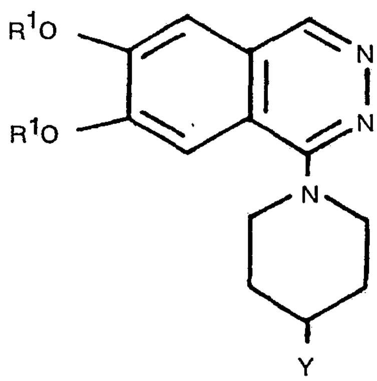

(I),

worin $R^1$ eine $C_1$—$C_4$-Alkylgruppe und Y eine Gruppe der Formel —$(CH_2)_m$—Z, worin m 1 oder 2 ist, und Z eine Gruppe, ausgewählt unter

—$OCONR^3R^4$,
—$N(R^2)COR^3$,
—$N(R^2)SO_2R^3$,
—$N(R^2)CONR^3R^4$,
—$N(R^2)SO_2NR^3R^4$,
und —$N(R^2)COOR^3$,

worin $R^2$ und $R^4$ jeweils unabhängig voneinander Wasserstoff oder $C_1$—$C_4$-Alkyl sind und $R^3$ $C_3$—$C_7$-Cycloalkyl ist, oder ein pharmazeutisch annehmbares Säureadditionssalz hiervon, gekennzeichnet durch die Umsetzung eines Phthalazins der Formel

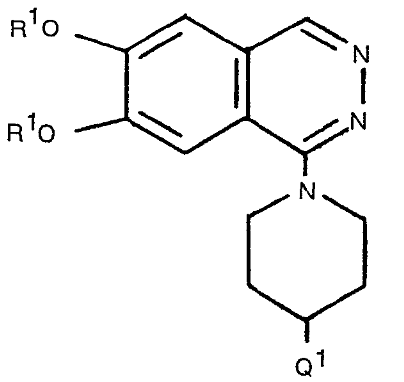

(III),

worin $R^1$ wie oben definiert ist und $Q^1$ eine leicht austretende Gruppe, wie Chlor, Brom, Jod, Niederalkoxy oder (Niederalkyl)-thio ist, mit einem Amin der Formel

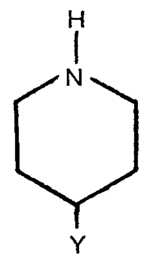

(IV),

worin Y wie oben definiert ist, und gegebenenfalls anschließende Umsetzung des so hergestellten Produkts der Formel (I) mit einer nicht-toxischen Säure zur Bildung eines pharmazeutisch annehmbaren Säureadditionssalzes.

2. Verfahren zur Herstellung eines Phthalazins der Formel

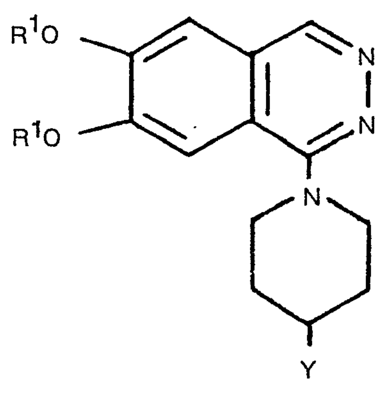

(I),

worin $R^1$ $C_1$—$C_4$-Alkyl und Y—$(CH_2)_mN(R^2)CONHR^3$, —$(CH_2)_mN(R^2)COR^3$ oder —$(CH_2)_mN(R^2)SO_2R_3$ ist, worin m 1 oder 2, $R^2$ $C_1$—$C_4$-Alkyl und $R^3$ $C_3$—$C_7$-Cycloalkyl ist, gekennzeichnet durch die Umsetzung eines Phthalazins der Formel

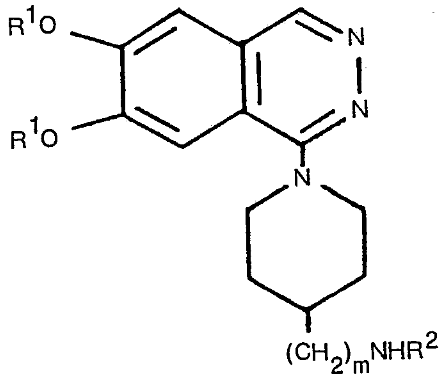

(V),

worin m, $R^1$ und $R^2$ wie oben definiert sind, mit, je nach Eignung, einer Verbindung der Formel $R^3X$ oder $(R^3CO)_2O$, worin X, je nach Eignung, —NCO, —COCl, —COBr, —$SO_2Cl$ oder —$SO_2Br$ ist, und gegebenenfalls durch anschließende Umsetzung des so hergestellten Produkts der Formel (I) mit einer nicht-toxischen Säure zur Bildung eines pharmazeutisch annehmbaren Säureadditionssalzes.

3. Verfahren nach Anspruch 1 oder 2, worin $R^1$ $CH_3$, $R^2$ H oder $CH_3$ und $R^3$ $C_3$—$C_6$-Cycloalkyl ist.

4. Verfahren nach Anspruch 1, worin $R^1$ $CH_3$ und Y unter —$CH_2CH_2N(CH_3)CON(CH_3)$-Cyclopentyl, —$CH_2CH_2N(CH_3)CO$-Cyclopentyl und —$CH_2CH_2N(CH_3)SO_2NH$-Cyclohexyl ausgewählt ist.

**Revendications pour l'Etat contractant AT**

1. Procédé de préparation d'une phtalazine de formule:

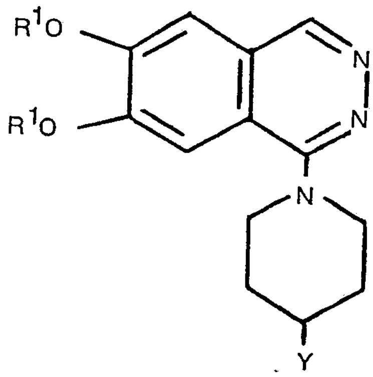

(I)

dans laquelle $R^1$ est un groupe alkyle en $C_1$ à $C_4$ est un groupe de formule: —$(CH_2)_m$—Z, où m est égal à 1 ou 2, et Z est un groupe choisi entre:

—$OCONR^3R^4$,
—$N(R^2)COR^3$,
—$N(R^2)SO_2R^3$,
—$N(R^2)CONR^3R^4$,
—$N(R^2)SO_2NR^3R^4$,
et —$N(R^2)COOR^3$,

où $R^2$ et $R^4$ représentent chacun, indépendamment, l'hydrogène ou un reste alkyle en $C_1$ à $C_4$ et $R^3$ est un reste cycloalkyle en $C_3$ à $C_7$; ou d'un sel d'addition d'acide pharmaceutiquement acceptable de ce composé, caractérisé en ce qu'il consiste à faire réagir une phtalazine de formule:

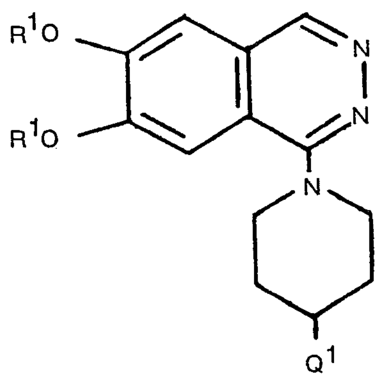

(III)

dans laquelle $R^1$ a la définition donnée ci-dessus et $Q^1$ est un groupe aisément partant tel que chloro, bromo, iodo, alkoxy inférieur (ou alkyle inférieur)thio, avec une amine de formule:

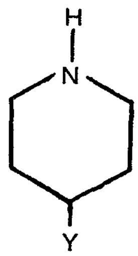

(IV)

dans laquelle Y a la définition donnée ci-dessus; puis, le cas échéant, à faire réagir le produit ainsi obtenu de formule (I) avec un acide non toxique pour former un sel d'addition d'acide pharmaceutiquement acceptable.

2. Procédé de préparation d'une phtalazine de formule:

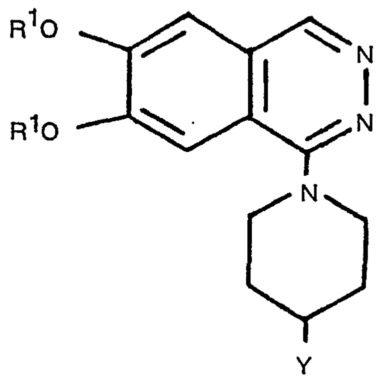

(I)

dans laquelle $R^1$ est un reste alkyle en $C_1$ à $C_4$ et Y est un reste $-(CH_2)_mN(R^2)CONHR^3$, $-(CH_2)_mN(R^2)COR^3$ ou $-(CH_2)_mN(R^2)SO_2R^3$, où m est égal à 1 ou 2, $R^2$ est un reste alkyle en $C_1$ à $C_4$ et $R^3$ est un reste cycloalkyle en $C_3$ à $C_7$, caractérisé par la réaction d'une phtalazine de formule:

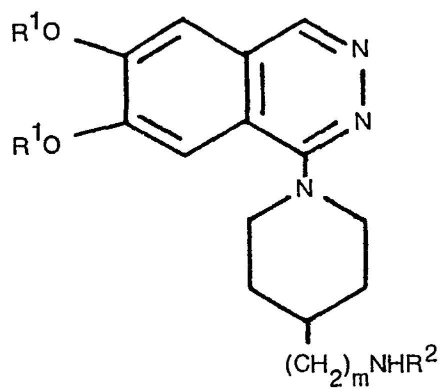

(V)

dans laquelle m, $R^1$ et $R^2$ ont la définition donnée ci-dessus, avec, selon le cas, un composé de formule $R^3X$ ou $(R^3CO)_2O$ où X est, selon le cas, un reste $-NCO$, $-COCl$, $-COBr$, $-SO_2Cl$ ou $-SO_2Br$; suivie le cas échéant de la réaction du produit ainsi obtenu de formule (I) avec un acide non toxique pour former un sel d'addition d'acide pharmaceutiquement acceptable.

3. Procédé suivant la revendication 1 ou 2, dans lequel $R^1$ est le groupe $CH_3$, $R^2$ est H ou le groupe $CH_3$ et $R^3$ est un groupe cycloalkyle en $C_3$ à $C_6$.

4. Procédé suivant la revendication 1, dans lequel $R^1$ est le groupe $CH_3$ et Y est choisi entre les groupes $-CH_2CH_2N(CH_3)CON(CH_3$.cyclopentyle, $-CH_2CH_2N(CH_3)CO$.cyclopentyle et $-CH_2CH_2N(CH_3)SO_2NH$.cyclohexyle.